# EUROPÄISCHE PATENTSCHRIFT

(11) **EP 0 720 980 B1**
(45) Veröffentlichungstag und Bekanntmachung des Hinweises auf die Patenterteilung: **26.04.2000**
(21) Anmeldenummer: 95120370.2
(22) Anmeldetag: 22.12.1995
(51) Int. Cl.: C07C 231/02, C07C 233/24, C07C 233/60

(54) **Verfahren zur Herstellung von Alkylcarbonsäure-4-Hydroxyaniliden**
Process for the preparation of 4-hydroxyanilides of alkylcarboxylic acid
Procédé pour la préparation de 4-hydroxyanilides d'acide alkylcarboxylique

(30) Priorität: 04.01.1995 DE 19500119
(43) Veröffentlichungstag der Anmeldung: 10.07.1996
(73) Patentinhaber: BAYER AG, 51368 Leverkusen (DE)
(72) Erfinder: Lantzsch, Reinhard, Dr., D-42115 Wuppertal (DE); Rivadeneira, Eric, Dr., D-51381 Leverkusen (DE); Lindner, Werner, Dr., D-51067 Köln (DE)

(56) Entgegenhaltungen:
- EP-A- 0 339 418
- EP-A- 0 569 792
- EP-A- 0 608 739
- GB-A- 1 255 161
- F. MÖLLER 'HOUBEN-WEYL, Methoden der organischen Chemie, Band XI/2' 1958 , GEORG THIEME VERLAG , STUTTGART * Seite 10 - Seite 14 ; Seite 13, Absatz 8 *
- CHEMICAL ABSTRACTS, vol. 92, no. 10, 10.März 1980 Columbus, Ohio, US; abstract no. 76952g, ISEITLIN, G. M. ET AL. 'Acylation in amide solvents' Seite 2; Spalte 1; & IZV. VYSSH. UCHEBN. ZAVED., KHIM. KHIM. TEKHNOL., Bd. 22, Nr. 10, 1979 Seiten 1190-1193,
- CHEMICAL ABSTRACTS, vol. 89, no. 13, 25.September 1978 Columbus, Ohio, US; abstract no. 107264d, IVANOVA, N. S. ET AL. 'Reactivity of hydroxyl group of aminophenols in reactions with benzoyl chloride in nonaqueous solutions.' Seite 729; Spalte 2; & IZV. VYSSH. UCHEBN. ZAVED., KHIM. KHIM. TEKHNOL., Bd. 21, Nr. 4, 1978 Seiten 529-533,

## Beschreibung

Die vorliegende Erfindung betrifft ein neues Verfahren zur Herstellung von bekannten Alkylcarbonsäure-4-Hydroxyaniliden.

Es ist bereits bekannt, daß Phenole, Aniline und o-Aminophenole mit carbonsäurehalogeniden umgesetzt werden können (Houben-Weyl, Methoden der org. chem., Band XI/2, 1958, Seite 4-14; C.Ab. vol. 92, no.10, 76952g: C.Ab., vol. 89, no. 13, 107264d). Alkylcarbonsäure-4-Hydroxyanilide durch Reaktion von 4-Hydroxyanilinen mit Alkylcarbonsäurehalogeniden erhalten werden können (EP-A 339418, GB-A-1255161). Die Herstellung ist nicht einfach, da sich mehrere Nebenprodukte bilden können. Reagiert das Carbonsäurechlorid zunächst mit der Hydroxygruppe des Aminophenols, so erhält man Carbonsäurephenylester statt der gewünschten Carbonsäureanilide. Weiterhin ist es möglich, daß die Aminogruppe des Aminophenols zweimal mit dem Carbonsäurechlorid reagiert, oder daß die Hydroxygruppe und die Aminogruppe des Aminophenols mit ähnlicher Geschwindigkeit mit dem Carbonsäurechlorid reagieren, so daß eine O,N-Bisacylverbindung oder eine O,N,N-Trisacylverbindung entsteht.

Bevorzugte Vorgehensweise in EP-A 339418 ist es deshalb, ein tertiäres Amin als Hilfsbase zu verwenden und bei Temperaturen zwischen 0 °C und 20 °C zu arbeiten. Außerdem sind mit Wasser mischbare Lösungsmittel bevorzugt. Dieses Vorgehen ist mit einer Vielzahl von Schwierigkeiten verbunden:
- Das in der Mutterlauge als Hydrochlorid vorliegende tertiäre Amin muß bei einem technischen Verfahren unter Einschaltung mehrerer Verfahrensschritte kostspielig zurückgewonnen werden.
- Die niedrige Reaktionstemperatur führt zu sehr langen Reaktionszeiten und zur Anwendung eines erheblichen Überschusses an Carbonsäurechlorid, so daß bereits erhebliche Mengen der zweifach oder dreifach acylierten Verbindung entstehen.
- Das Lösungsmittel ist aus der wäßrigen Mutterlauge nur unter erhöhtem Destillationsaufwand zurückzugewinnen.
- Die wäßrige Mutterlauge stellt von der Menge und Belastung her ein erhebliches Abwasserproblem dar.
- Erst nach Umkristallisieren erhält man ein Produkt ausreichender Reinheit.

Ein verbessertes Verfahren ist in der EP-A 569792 beschrieben: es wird in einem Zweiphasensystem aus Wasser und einem organischen Lösungsmittel gearbeitet und als Base bevorzugt eine wäßrige Base, wie z. B. 20 % ige Natronlauge, eingesetzt.

Das Verfahren hat den Nachteil, daß solche Zweiphasensysteme, in denen weder Ausgangsprodukt noch Endprodukt löslich sind, schlecht rührbar sind. Ferner fällt eine große Menge Chlorverbindungen enthaltendes Abwasser an, das nicht ohne weiteres biologisch abbaubar ist.

Es wurde nun gefunden, daß man Alkylcarbonsäure-4-Hydroxyanilide der allgemeinen Formel (I) 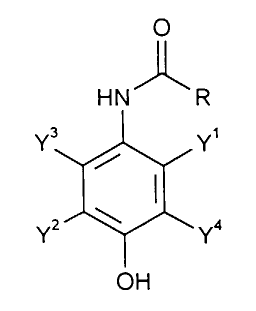 in welcher
- R: für gegebenenfalls einfach oder mehrfach, gleich oder verschieden durch Halogen substituiertes Alkyl oder für gegebenenfalls einfach oder mehrfach, gleich oder verschieden durch Alkyl, Halogenalkyl oder Halogen substituiertes Cycloalkyl steht,
- Y¹, Y², Y³, Y⁴: gleich oder verschieden sind und für Wasserstoff, Halogen, Cyano, jeweils gegebenenfalls halogensubstituiertes Alkyl, Alkoxy oder Alkylthio stehen,
in hoher Ausbeute und hoher Reinheit erhält,
wenn man Aminophenole der allgemeinen Formel (II) 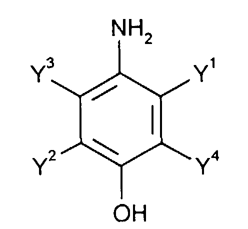 in welcher
- Y¹, Y², Y³, Y⁴: die oben angegebene Bedeutung haben,
mit Carbonsäurederivaten der allgemeinen Formel (III) 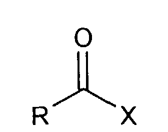 in welcher
- R: die oben angegebene Bedeutung hat und
- X: für Halogen, vorzugsweise Chlor steht,
ohne Basenzusatz in einem organischen Lösungsmittel in Abwesenheit von Wasser bei Temperaturen von 50 bis 180 °C umsetzt.

In den Definitionen sind die Kohlenwasserstoffketten, wie Alkyl, auch in Verknüpfung mit Heteroatomen, wie in Alkoxy oder Alkylthio, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Das erfindungsgemäße Verfahren weist eine Reihe von Vorteilen auf: Die acylierten Aminophenole der allgemeinen Formel (I) entstehen in sehr hoher Ausbeute, es wird kein Abwasser gebildet und der freiwerdende Halogenwasserstoff, vorzugsweise Chlorwasserstoff, entweicht gasförmig und kann leicht wieder verwendet werden. Die Produktreinheiten liegen über 95 %.

Es ist als ausgesprochen überraschend zu bezeichnen, daß gemäß dem erfindungsgemäßen Verfahren so gute Ausbeuten erzielt werden, da unter den Reaktionsbedingungen sowohl Aniline oder Anilinhydrochloride (Houben-Weyl, Band E5, Seite 972) mit Säurechloriden reagieren, Phenole aber bereits unter sehr milden Bedingungen ohne Säurefänger zu Estern reagieren (Houben-Weyl, Band E5, Seite 543).

Es war daher vom Fachmann nicht zu erwarten, daß die Herstellung der Verbindungen der allgemeinen Formel (I) nach dieser Methode in hoher Ausbeute und ausreichender Reinheit erfolgen würde.

Nach dem erfindungsgemäßen Verfahren werden vorzugsweise Verbindungen der allgemeinen Formel (I) hergestellt,
in welcher
- R: für gegebenenfalls einfach bis vierfach, gleich oder verschieden durch Halogen substituiertes, geradkettiges oder verzweigtes Alkyl mit 1 - 8 Kohlenstoffatomen, oder für jeweils gegebenenfalls einfach bis vierfach, gleich oder verschieden durch Halogen, geradkettiges oder verzweigtes Halogenalkyl oder Alkyl mit 1 bis 4 Kohlenstoffatomen substituiertes Cyclopropyl, Cyclobutyl, Cyclopentyl, Cyclohexyl oder Cycloheptyl steht,
- Y¹: für Wasserstoff, Fluor, Chlor, Brom, Cyano, geradkettiges oder verzweigtes Alkyl mit mit 1 bis 4 Kohlenstoffatomen, jeweils geradkettiges oder verzweigtes Alkoxy oder Alkylthio mit je 1 bis 4 Kohlenstoffatomen oder für jeweils geradkettiges oder verzweigtes Halogenalkyl, Halogenalkoxy, Halogenalkylthio mit 1 bis 4 Kohlenstoffatomen und mit 1 bis 5 gleichen oder verschiedenen Halogenatomen steht,
- Y²: für Wasserstoff, Fluor, Chlor, Brom, Cyano, geradkettiges oder verzweigtes Alkyl mit mit 1 bis 4 Kohlenstoffatomen, jeweils geradkettiges oder verzweigtes Alkoxy oder Alkylthio mit je 1 bis 4 Kohlenstoffatomen oder für jeweils geradkettiges oder verzweigtes Halogenalkyl, Halogenalkoxy, Halogenalkylthio mit 1 bis 4 Kohlenstoffatomen und mit 1 bis 5 gleichen oder verschiedenen Halogenatomen steht,
- Y³: für Wasserstoff, Fluor, Chlor, Brom, Cyano, geradkettiges oder verzweigtes Alkyl mit mit 1 bis 4 Kohlenstoffatomen, jeweils geradkettiges oder verzweigtes Alkoxy oder Alkylthio mit je 1 bis 4 Kohlenstoffatomen oder für jeweils geradkettiges oder verzweigtes Halogenalkyl, Halogenalkoxy, Halogenalkylthio mit 1 bis 4 Kohlenstoffatomen und mit 1 bis 5 gleichen oder verschiedenen Halogenatomen steht,
- Y⁴: für Wasserstoff, Fluor, Chlor, Brom, Cyano, geradkettiges oder verzweigtes Alkyl mit mit 1 bis 4 Kohlenstoffatomen, jeweils geradkettiges oder verzweigtes Alkoxy oder Alkylthio mit je 1 bis 4 Kohlenstoffatomen oder für jeweils geradkettiges oder verzweigtes Halogenalkyl, Halogenalkoxy, Halogenalkylthio mit 1 bis 4 Kohlenstoffatomen und mit 1 bis 5 gleichen oder verschiedenen Halogenatomen steht.

Nach dem erfindungsgemäßen Verfahren werden insbesondere Verbindungen der allgemeinen Formel (I) hergestellt,
in welcher
- R: für jeweils gegebenenfalls einfach, zweifach oder dreifach, gleich oder verschieden durch Fluor, Chlor oder Brom substituiertes Methyl, Ethyl, n- oder i-Propyl, n-, i-, sec-, oder t-Butyl, 1-, 2-, 3-, tert-, oder neo-Pentyl, 2-Methyl-1-butyl, 3-Methyl-1-butyl, 3-Methyl-2-butyl, oder für jeweils gegebenenfalls einfach oder zweifach, gleich oder verschieden durch Fluor, Chlor, Brom, Methyl, Ethyl, n-Propyl, i-Propyl, Chlormethyl, Chlorethyl, oder Trifluormethyl substituiertes Cyclopropyl, Cyclobutyl, Cyclopentyl, Cyclohexyl oder Cycloheptyl steht,
- Y¹: für Wasserstoff, Fluor, Chlor, Brom, Cyano, Methyl oder Trifluormethyl steht,
- Y²: für Wasserstoff, Fluor, Chlor, Brom, Cyano, Methyl oder Trifluormethyl steht,
- Y³: für Wasserstoff, Fluor, Chlor, Brom, Cyano, Methyl oder Trifluormethyl steht,
- Y⁴: für Wasserstoff, Fluor, Chlor, Brom, Cyano, Methyl oder Trifluormethyl steht.

Ganz besonders bevorzugt werden nach dem erfindungsgemäßen Verfahren Verbindungen der allgemeinen Formel (I) hergestellt,
in welcher
- R: für jeweils gegebenenfalls einfach, zweifach oder dreifach, gleich oder verschieden durch Fluor, Chlor oder Brom substituiertes Methyl, Ethyl, n-oder i-Propyl, n-, i-, sec-, oder t-Butyl, 1-, 2-, 3-, tert-, oder neo-Pentyl, 2-Methyl-1-butyl, 3-Methyl-1-butyl, 3-Methyl-2-butyl, oder für jeweils in 1 Stellung durch Methyl, Ethyl, Chlor, Brom oder Trifluormethyl substituiertes Cyclopropyl, Cyclobutyl, Cyclopentyl, Cyclohexyl oder Cycloheptyl steht, das jeweils gegebenenfalls durch einen weiteren Alkylrest mit 1, 2 oder 3 Kohlenstoffatomen substituiert ist steht,
- Y¹: für Wasserstoff, Fluor, Chlor, Brom, Cyano, Methyl oder Trifluormethyl steht,
- Y²: für Wasserstoff, Fluor, Chlor, Brom, Cyano, Methyl oder Trifluormethyl steht,
- Y³: für Wasserstoff, Fluor, Chlor, Brom, Cyano, Methyl oder Trifluormethyl steht,
- Y⁴: für Wasserstoff, Fluor, Chlor, Brom, Cyano, Methyl oder Trifluormethyl steht.

Die zur Durchführung des erfindungsgemäßen Verfahrens als Ausgangsstoffe benötigten Aminophenole sind durch die Formel (II) allgemein definiert. In dieser allgemeinen Formel (II) haben die Reste Y¹, Y², Y³ und Y⁴ vorzugsweise bzw. insbesondere diejenigen Bedeutungen, die bereits oben im Zusammenhang mit der Beschreibung der Verbindungen der allgemeinen Formel (I) als bevorzugt bzw. als insbesondere bevorzugt für Y¹, Y², Y³ und Y⁴ angegeben wurden. Die Ausgangsstoffe der allgemeinen Formel (II) sind bekannt und/oder können nach an sich bekannten Verfahren hergestellt werden (vergleiche Cesare Ferri, "Reaktionen in der organischen Synthese", 1978, 81, 89, 91, 97, 118, 120, 122, 124, 126, 128; EP-A 339418).

Die zur Durchführung des erfindungsgemäßen Verfahrens außerdem als Ausgangsstoffe benötigten Alkylcarbonsäurederivate sind durch die Formel (III) allgemein definiert. In dieser Formel (III) hat der Rest R vorzugsweise bzw. insbesondere diejenige Bedeutung, die bereits oben im Zusammenhang mit der Beschreibung der Verbindungen der allgemeinen Formel (I) als bevorzugt bzw. als insbesondere bevorzugt für R angegeben wurde. X steht für Fluor, Chlor, Brom oder Iod, bevorzugt Chlor. Die Ausgangsstoffe der allgemeinen Formel (III) sind bekannt und/oder können nach an sich bekannten Verfahren hergestellt werden (vergleiche Diversi et. al., Synthesis 1971, 258; US-A 3674831; Cesare Ferri, "Reaktionen in der organischen Synthese", 1978, 460-461, Georg Thieme Verlag, Stuttgart).

Als Verdünnungsmittel zur Durchführung des erfindungsgemäßen Verfahrens kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin, bevorzugt Toluol oder Xylol; halogenierte Kohlenwasserstoffe, wie beispielsweise Chlorbenzol, Dichlorbenzol, Dichlormethan, Chloroform, Tetrachlormethan, Dichlorethan oder Trichlorethan, bevorzugt Chlorbenzol oder Dichlorbenzol; Ether, wie beispielsweise Diethylether, Diisopropylether, Methyl-t-butylether, Methyl-t-Amylether, Dioxan, Tetrahydrofuran, 1,2- Dimethoxyethan, 1,2-Diethoxyethan oder Anisol, bevorzugt 1,2-Dimethoxyethan, Methyl-t-butylether oder Methyl-t-Amylether; sowie Nitrile, wie beispielsweise Acetonitril, Propionitril, n- oder iso-Butyronitril oder Benzonitril, bevorzugt Acetonitril, sowie n-oder iso-Butyronitril.

Die benutzte Reaktionstemperaturen Sind von 60°C bis 180°C, vorzugsweise Temperaturen von 70°C bis 150°C.

Das erfindungsgemäße Verfahren wird im allgemeinen unter Normaldruck oder vermindertem Druck durchgeführt. Es ist jedoch auch möglich, unter erhöhtem Druck zu arbeiten, wobei aber durch zeitweiliges Entspannen der Apparatur der gebildete Halogenwasserstoff entfernt werden muß. Vorzugsweise arbeitet man bei Normaldruck, gegebenenfalls wird ein Inertgas, wie z. B. Stickstoff, durch die Reaktionslösung geleitet, um den gebildeten Halogenwasserstoff vollständig zu entfernen.

Zur Durchführung des erfindungsgemäßen Verfahrens zur Herstellung der Verbindungen der Formel (I) setzt man pro Mol des Aminophenols der allgemeinen Formel (II) im allgemeinen 1 bis 1,3 Mol, vorzugsweise 1 bis 1,2 Mol, des Cycloalkancarbonsäurederivates der allgemeinen Formel (III) ein.

In einer möglichen Ausführungsform wird das Cycloalkancarbonsäurederivat der allgemeinen Formel (III) in dem organischen Lösungsmittel vorgelegt und erwärmt. Anschließend wird das Aminophenol der allgemeinen Formel (II) bei Temperaturen zwischen 80°C und 120°C zudosiert. Zur Vervollständigung der Reaktion kann gegebenenfalls auch noch höher erhitzt werden.

In einer bevorzugten Ausführungsform wird das Aminophenol der allgemeinen Formel (II) in dem organischen Lösungsmittel vorgelegt und erwärmt. Anschließend wird das Cycloalkancarbonsäurederivat der allgemeinen Formel (III) bei Temperaturen zwischen 80°C und 120°C zudosiert. Zur Vervollständigung der Reaktion kann gegebenenfalls auch noch höher erhitzt werden.

Die erfindungsgemäß hergestellten Stoffe können gegen Pflanzenschädlinge, insbesondere phytopathogene Pilze, eingesetzt werden.

### Herstellungsbeispiele

### Beispiel 1

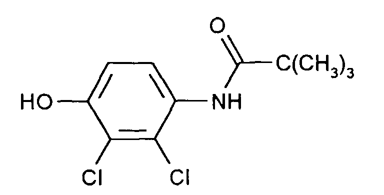

9,3 g (0,05 mol) 4-Amino-2,3-dichlorphenol (Gehalt: 96 %) werden in 175 ml Toluol suspendiert und unter Rühren auf 100°C erhitzt. Dann tropft man 6,3 g (0,0525 mol) Pivalinsäurechlorid bei 100 - 105°C zu und rührt nach beendeter Zugabe weitere 4 Stunden unter Rückfluß. Nach Abkühlen der Reaktionsmischung wird der Niederschlag bei 0 - 10°C abfiltriert und mit wenig Toluol gewaschen. Man erhält 12,5 g (95,4 % der Theorie) Pivalinsäure-(2,3-dichlor-4-hydroxy)-anilid vom Schmelzpunkt 62°C.

### Beispiel 2

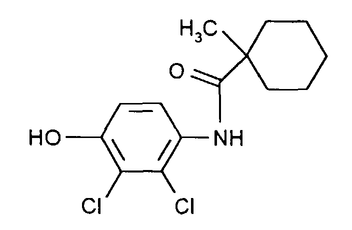

9,3 g (0,05 mol) 4-Amino-2,3-dichlorphenol (Gehalt: 96 %) werden in 175 ml Toluol suspendiert und unter Rühren auf 100°C erhitzt. Dann tropft man 8,8 g (0,0525 mol) 1-Methylcyclohexan-1-carbonsäurechlorid (Gehalt: 96 %) bei 100 - 105°C zu und rührt nach beendeter Zugabe weitere 4 Stunden unter Rückfluß. Nach Abkühlen der Reaktionsmischung wird der Niederschlag bei 0 - 10°C abfiltriert und mit wenig Toluol gewaschen. Man erhält 13,95 g (92,3 % der Theorie) 1-Methylcyclohexan-1-carbonsäure-(2,3-dichlor-4-hydroxy)-anilid vom Schmelzpunkt 154°C.

### Beispiel 3

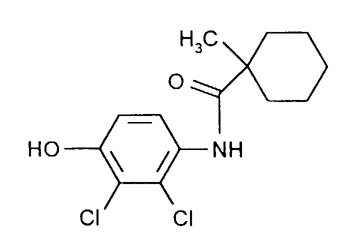

9,3 g (0,05 mol) 4-Amino-2,3-dichlorphenol (Gehalt: 96 %) werden in 50 ml 1,2-Dimethoxyethan gelöst und unter Rühren auf 88°C erhitzt. Innerhalb von 20 Minuten tropft man 8,8 g (0,0525 mol) 1-Methylcyclohexan-1-carbonsäurechlorid (Gehalt: 96 %) bei 100 - 105°C zu und rührt nach beendeter Zugabe weitere 6 Stunden unter Rückfluß. Nach dem Abkühlen der Reaktionsmischung filtriert man 1,5 g 4-Amino-2,3-dichlorphenol-Hydrochlorid ab. Das Filtrat wird eingeengt, mit wenig Toluol ausgerührt und abfiltriert. Man erhält 13,95 g 1-Methylcyclohexan-1-carbonsäure-(2,3-dichlor-4-hydroxy)-anilid vom Schmelzpunkt 154°C. Dies entspricht einer Ausbeute von 90,8 % der Theorie, bezogen auf umgesetztes 4-Amino-2,3-dichlorphenol.

## Patentansprüche

1. Verfahren zur Herstellung von Verbindungen der allgemeinen Formel (I) 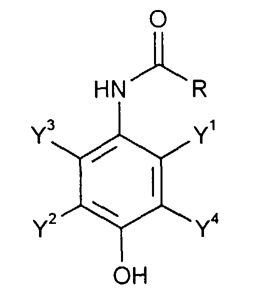 in welcher
R für gegebenenfalls einfach oder mehrfach, gleich oder verschieden durch Halogen substituiertes Alkyl oder für gegebenenfalls einfach oder mehrfach, gleich oder verschieden durch Alkyl, Halogenalkyl oder Halogen substituiertes Cycloalkyl steht,
Y¹, Y², Y³, Y⁴ gleich oder verschieden sind und für Wasserstoff, Halogen, Cyano, jeweils gegebenenfalls halogensubstituiertes Alkyl, Alkoxy oder Alkylthio stehen,
dadurch gekennzeichnet, daß man Aminophenole der allgemeinen Formel (II) 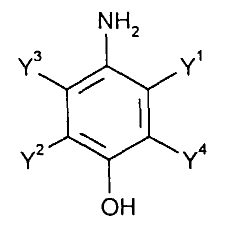 in welcher
Y¹, Y², Y³, Y⁴ die oben angegebene Bedeutung haben,
mit Carbonsäurederivaten der allgemeinen Formel (III) 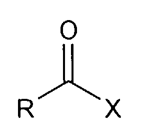 in welcher
R die oben angegebene Bedeutung hat und
X für Halogen, vorzugsweise Chlor steht.
ohne Basenzusatz in einem organischen Lösungsmittel in Abwesenheit von Wasser bei Temperaturen von 50-180°C umsetzt.

2. Verfahren gemäß Anspruch 1, dadurch gekennzeichnet, daß Verbindungen der allgemeinen Formel (I) hergestellt werden,
in welcher
R für gegebenenfalls einfach bis vierfach, gleich oder verschieden durch Halogen substituiertes, geradkettiges oder verzweigtes Alkyl mit 1 - 8 Kohlenstoffatomen, oder für jeweils gegebenenfalls einfach bis vierfach, gleich oder verschieden durch Halogen, geradkettiges oder verzweigtes Halogenalkyl oder Alkyl mit 1 bis 4 Kohlenstoffatomen substituiertes Cyclopropyl, Cyclobutyl, Cyclopentyl, Cyclohexyl oder Cycloheptyl steht,
Y¹ für Wasserstoff, Fluor, Chlor, Brom, Cyano, geradkettiges oder verzweigtes Alkyl mit mit 1 bis 4 Kohlenstoffatomen, jeweils geradkettiges oder verzweigtes Alkoxy oder Alkylthio mit je 1 bis 4 Kohlenstoffatomen oder für jeweils geradkettiges oder verzweigtes Halogenalkyl, Halogenalkoxy, Halogenalkylthio mit 1 bis 4 Kohlenstoffatomen und mit 1 bis 5 gleichen oder verschiedenen Halogenatomen steht,
Y² für Wasserstoff, Fluor, Chlor, Brom, Cyano, geradkettiges oder verzweigtes Alkyl mit mit 1 bis 4 Kohlenstoffatomen, jeweils geradkettiges oder verzweigtes Alkoxy oder Alkylthio mit je 1 bis 4 Kohlenstoffatomen oder für jeweils geradkettiges oder verzweigtes Halogenalkyl, Halogenalkoxy, Halogenalkylthio mit 1 bis 4 Kohlenstoffatomen und mit 1 bis 5 gleichen oder verschiedenen genatomen steht,
Y³ für Wasserstoff, Fluor, Chlor, Brom, Cyano, geradkettiges oder verzweigtes Alkyl mit mit 1 bis 4 Kohlenstoffatomen, jeweils geradkettiges oder verzweigtes Alkoxy oder Alkylthio mit je 1 bis 4 Kohlenstoffatomen oder für jeweils geradkettiges oder verzweigtes Halogenalkyl, Halogenalkoxy, Halogenalkylthio mit 1 bis 4 Kohlenstoffatomen und mit 1 bis 5 gleichen oder verschiedenen Halogenatomen steht,
Y⁴ für Wasserstoff, Fluor, Chlor, Brom, Cyano, geradkettiges oder verzweigtes Alkyl mit mit 1 bis 4 Kohlenstoffatomen, jeweils geradkettiges oder verzweigtes Alkoxy oder Alkylthio mit je 1 bis 4 Kohlenstoffatomen oder für jeweils geradkettiges oder verzweigtes Halogenalkyl, Halogenalkoxy, Halogenalkylthio mit 1 bis 4 Kohlenstoffatomen und mit 1 bis 5 gleichen oder verschiedenen Halogenatomen steht.

3. Verfahren gemäß Anspruch 1, dadurch gekennzeichnet, daß das Verfahren bei Reaktionstemperaturen von 70°C bis 150°C durchgeführt wird.

4. Verfahren gemäß Anspruch 1, dadurch gekennzeichnet, daß man pro Mol des Aminophenols der allgemeinen Formel (II) im allgemeinen 1 bis 1,3 Mol des Cycloalkancarbonsäurederivates der allgemeinen Formel (III) ein setzt.

## Claims

1. Process for the preparation of compounds of the general formula (I) 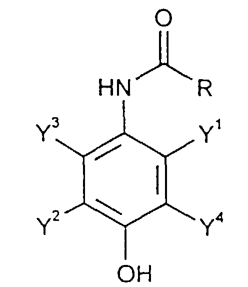 in which
R represents alkyl which is optionally mono- or polysubstituted in an identical or different manner by halogen, or represents cycloalkyl which is optionally mono- or polysubstituted in an identical or different manner by alkyl, halogenoalkyl or halogen and
Y¹, Y², Y³, Y⁴ are identical or different and represent hydrogen, halogen, cyano, in each case optionally halogen-substituted alkyl, alkoxy or alkylthio,
characterized in that aminophenols of the general formula (II) 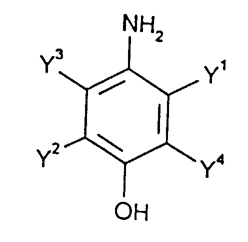 in which
Y¹, Y², Y³, Y⁴ have the abovementioned meaning,
with carboxylic acid derivatives of the general formula (III) 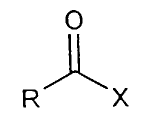 in which
R has the abovementioned meaning and
X represents halogen, preferably chlorine,
without the addition of a base in an organic solvent in the absence of water at temperatures of 50-180°C.

2. Process according to Claim 1, characterized in that compounds of the general formula (I)
in which
R represents straight-chain or branched alkyl having 1 - 8 carbon atoms which is optionally mono- to tetrasubstituted in an identical or different manner by halogen, or represents cyclopropyl, cyclobutyl, cyclopentyl, cyclohexyl or cycloheptyl which are in each case optionally mono- to tetrasubstituted in an identical or different manner by halogen, straight-chain or branched halogenoalkyl or alkyl having 1 to 4 carbon atoms,
Y¹ represents hydrogen, fluorine, chlorine, bromine, cyano, straight-chain or branched alkyl having 1 to 4 carbon atoms, or in each case straight-chain or branched alkoxy or alkylthio having in each case 1 to 4 carbon atoms, or represents in each case straight-chain or branched halogenoalkyl, halogenoalkoxy or halogenoalkylthio having 1 to 4 carbon atoms and 1 to 5 identical or different halogen atoms,
Y² represents hydrogen, fluorine, chlorine, bromine, cyano, straight-chain or branched alkyl having 1 to 4 carbon atoms, or in each case straight-chain or branched alkoxy or alkylthio having in each case 1 to 4 carbon atoms, or represents in each case straight-chain or branched halogenoalkyl, halogenoalkoxy or halogenoalkylthio having 1 to 4 carbon atoms and 1 to 5 identical or different halogen atoms,
Y³ represents hydrogen, fluorine, chlorine, bromine, cyano, straight-chain or branched alkyl having 1 to 4 carbon atoms, or in each case straight-chain or branched alkoxy or alkylthio having in each case 1 to 4 carbon atoms, or represents in each case straight-chain or branched halogenoalkyl, halogenoalkoxy or halogenoalkylthio having 1 to 4 carbon atoms and 1 to 5 identical or different halogen atoms,
Y⁴ represents hydrogen, fluorine, chlorine, bromine, cyano, straight-chain or branched alkyl having 1 to 4 carbon atoms, or in each case straight-chain or branched alkoxy or alkylthio having in each case 1 to 4 carbon atoms, or atoms, or represents in each case straight-chain or branched halogenoalkyl, halogenoalkoxy or halogenoalkylthio having 1 to 4 carbon atoms and 1 to 5 identical or different halogen atoms,
are prepared.

3. Process according to Claim 1, characterized in that the process is carried out at reaction temperatures from 70°C to 150°C.

4. Process according to Claim 1, characterized in that in general 1 to 1.3 mol of the cycloalkanecarboxylic acid derivative of the general formula (III) are employed per mol of the aminophenol of the general formula (II).

## Revendications

1. Procédé pour la préparation de composés répondant à la formule générale (I) 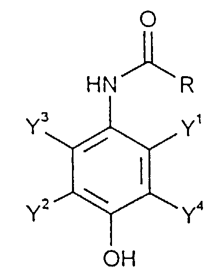 dans laquelle
R représente un groupe alkyle portant le cas échéant un ou plusieurs substituants halogéno identiques ou différents, ou représente un groupe cycloalkyle portant le cas échéant un ou plusieurs substituants identiques ou différents alkyle, halogénalkyle ou halogéno,
Y¹, Y², Y³, Y⁴ sont identiques ou différents et représentent un atome d'hydrogène, un atome d'halogène, un groupe cyano, un groupe alkyle, un groupe alcoxy ou un groupe alkylthio, chacun des trois groupes mentionnés en dernier lieu portant le cas échéant un ou plusieurs substituants halogéno identiques ou différents,
caractérisé en ce qu'on fait réagir des aminophénols répondant à la formule générale (II) 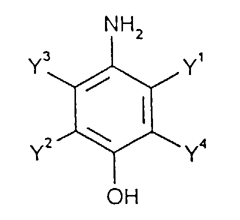 dans laquelle
Y¹, Y², Y³, Y⁴ ont la signification indiquée ci-dessus,
avec des dérivés d'acides carboxyliques répondant à la formule générale (III) 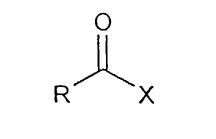 dans laquelle
R a la signification indiquée ci-dessus, et
X représente un atome d'halogène, de préférence un atome de chlore,
sans addition de bases, dans un solvant organique, en l'absence d'eau, à des températures de 50 à 180°C.

2. Procédé selon la revendication 1, caractérisé en ce qu'on prépare des composés répondant à la formule (I)
dans laquelle
R représente un groupe alkyle à chaîne droite ou ramifiée contenant de 1 à 8 atomes de carbone, portant le cas échéant de un à quatre substituants halogéno identiques ou différents, ou représente un groupe cyclopropyle, un groupe cyclobutyle, un groupe cyclopentyle, un groupe cyclohexyle ou un groupe cycloheptyle, chacun de ces groupes portant le cas échéant de un à quatre substituants identiques ou différents halogéno, halogénalkyle ou alkyle à chaîne droite ou ramifiée contenant de 1 à 4 atomes de carbone,
Y¹ représente un atome d'hydrogène, un atome de fluor, un atome de chlore, un atome de brome, un groupe cyano, un groupe alkyle à chaîne droite ou ramifiée contenant de 1 à 4 atomes de carbone, un groupe alcoxy ou un groupe alkylthio respectivement à chaîne droite ou ramifiée et contenant respectivement de 1 à 4 atomes de carbone, ou représente un groupe halogénalkyle, un groupe halogénalcoxy, un groupe halogénalkylthio respectivement à chaîne droite ou ramifiée, contenant de 1 à 4 atomes de carbone et de 1 à 5 atomes d'halogène identiques ou différents,
Y² représente un atome d'hydrogène, un atome de fluor, un atome de chlore, un atome de brome, un groupe cyano, un groupe alkyle à chaîne droite ou ramifiée contenant de 1 à 4 atomes de carbone, un groupe alcoxy ou un groupe alkylthio respectivement à chaîne droite ou ramifiée contenant respectivement de 1 à 4 atomes de carbone, ou représente un groupe halogénalkyle, un groupe halogénalcoxy, un groupe halogénalkylthio respectivement à chaîne droite ou ramifiée contenant de 1 à 4 atomes de carbone et de 1 à 5 atomes d'halogène identiques ou différents,
Y³ représente un atome d'hydrogène, un atome de fluor, un atome de chlore, un atome de brome, un groupe cyano, un groupe alkyle à chaîne droite ou ramifiée contenant de 1 à 4 atomes de carbone, un groupe alcoxy ou un groupe alkylthio respectivement à chaîne droite ou ramifiée contenant respectivement de 1 à 4 atomes de carbone, ou représente un groupe halogénalkyle, un groupe halogénalcoxy, un groupe halogénalkylthio respectivement à chaîne droite ou ramifiée contenant de 1 à 4 atomes de carbone et de 1 à 5 atomes d'halogène identiques ou différents,
Y⁴ représente un atome d'hydrogène, un atome de fluor, un atome de chlore, un atome de brome, un groupe cyano, un groupe alkyle à chaîne droite ou ramifiée contenant de 1 à 4 atomes de carbone, un groupe alcoxy ou un groupe alkylthio respectivement à chaîne droite ou ramifiée contenant respectivement de 1 à 4 atomes de carbone, ou représente un groupe halogénalkyle, un groupe halogénalcoxy, un groupe halogénalkylthio respectivement à chaîne droite ou ramifiée contenant de 1 à 4 atomes de carbone et de 1 à 5 atomes d'halogène identiques ou différents.

3. Procédé selon la revendication 1, caractérisé en ce qu'on effectue le procédé à des températures réactionnelles de 70°C à 150°C.

4. Procédé selon la revendication 1, caractérisé en ce qu'on met en oeuvre, par mole de l'aminophénol répondant à la formule générale (II), en général de 1 à 1,3 mole du dérivé d'acide cycloalcane-carboxylique répondant à la formule générale (III).
